# EUROPÄISCHE PATENTSCHRIFT

(11) **EP 3 072 492 B1**
(45) Veröffentlichungstag und Bekanntmachung des Hinweises auf die Patenterteilung: **29.07.2020**
(21) Anmeldenummer: 16000704.3
(22) Anmeldetag: 24.03.2016
(51) Int. Cl.: A61H 1/00, A61H 23/02, E04C 2/34

(54) **THERAPIEVORRICHTUNG MIT EINER FEDERGELAGERTEN THERAPIEPTATTE UND UNWUCHTMOTOREN**
THERAPY DEVICE
DISPOSITIF DE TRAITEMENT

(30) Priorität: 26.03.2015 DE 102015003854
(43) Veröffentlichungstag der Anmeldung: 28.09.2016
(73) Patentinhaber: Katterfeld, Hagen, 79098 Freiburg (DE)
(72) Erfinder: Katterfeld, Hagen, 79098 Freiburg (DE)
(74) Vertreter: mepat Patentanwälte

(56) Entgegenhaltungen:
- EP-A1- 1 772 135
- WO-A1-2005/077654
- DE-A1-102011 117 680
- DE-U1- 20 217 341
- DE-U1-202014 001 777
- GB-A- 1 065 601
- US-A- 1 914 646
- US-A1- 2008 250 564
- US-A1- 2014 323 816
- Anonymous: "Dual-Fliehkraft-Rüttelmotoren DFA 10", , 1. April 2012 (2012-04-01), XP055282195, Eibenstock Gefunden im Internet: URL:https://web.archive.org/web/2012040109 3612/http://www.eviro.com/dfa-10.html [gefunden am 2016-06-21]

## Beschreibung

Die Erfindung betrifft eine Therapievorrichtung.

Es sind Therapievorrichtungen mit vibrierenden Therapieflächen bekannt, insbesondere mit solchen, die in Normalenrichtung oszillieren. Solche Vorrichtungen werden als Trainingsgerät für Sportler sowie in der Physiotherapie zum Muskeltraining eingesetzt, da die Einwirkung der Vibration oder Oszillation vom Körper eine Kompensationsbewegung verlangt, was sich als Trainingseffekt bemerkbar macht.

Es wird aber auch angenommen, dass die Behandlung mit einer oszillierenden Bewegung in einem niederen Frequenzbereich von ca. 5 bis 100 Hz (niederfrequente Stoßwellentherapie) eine physiologische Wirkung auf Zellebene hat und den Zellstoffwechsel anregt, mögliche Blockaden löst und die Körpersysteme wieder ins Gleichgewicht bringt, was zu einer Revitalisierung des gesamten Organismus beitragen kann. Diese Wirkung ist umso besser, je weniger "Störanteile" die Oszillationsbewegung in Richtungen hat, die von der Normalenrichtung abweichen.

Es werden daher bei bekannten Therapievorrichtungen technische Vorkehrungen getroffen, um eine Bewegung der Therapieplatte in einer von der Normalen abweichende Richtung zu unterbinden. Aus dem Stand der Technik ist dazu beispielsweise bekannt, die Therapieplatte auf Linearführungen zu führen, was meist ein erhebliches Radialspiel in den Führungen mit sich bringt, das zu den von der Normalenrichtung abweichenden Bewegungen führt, die im Stand der Technik nicht oder nicht ausreichend kompensiert werden, weil sie dort offenbar auch nicht als so störend angesehen werden. Eine solche Vorrichtung ist beispielsweise aus DE 10 2011 117 680 A1 oder aus WO 2006 001656 A1 bekannt.

Auch die US 2643651 A offenbart eine federgelagerte Therapieplatte, die elektromotorisch angetrieben ist und Oszillationsbewegungen ausführt, die allerdings auch Richtungen haben, die von der Normalenrichtung abweichen.

Der motorische Antrieb von Ganzkörpervibrationsgeräten ist auch Gegenstand der DE 10 2007 024 835 A1, die hierzu einen Linearmotor offenbart.

Dazu offenbart die DE 202 17 341 U1 eine Therapievorrichtung die die Erzeugung und Übertragung von mechanischen, rhythmischen Schwingungen auf unterschiedliche Teile des menschlichen Körpers beschreibt und eine Schwingplatte hat, die die mechanischen Schwingungen auf die entsprechenden Teile des menschlichen Körpers überträgt, die in etwa in senkrechter Richtung bezogen auf ihre Kontaktoberfläche gleichförmig schwingt. Ein Antrieb mit zwei nebeneinander symmetrisch angeordneten und fest mit der Schwingplatte verbundene elektrischen Vibrationsmotoren haben, deren jeweils zwei auf den beiden Enden der Antriebswelle der Motoren aufgebrachten Unwuchtmassen mit gleichem Massenträgheitsmoment synchron aber in entgegengesetzter Drehrichtung drehen, sorgt für die Schwingung der Platte, die gedämpft gelagert ist.

Ein weiterer Ansatz geht von zwei gegenläufig rotierenden Unwuchtmotoren aus, die so betrieben werden, dass sie phasensynchron laufen, sodass sich die Bewegungsanteile in Ebenenrichtung der Therapieplatte aufheben und die Bewegungsanteile in Normalenrichtung addiert werden. Dieses Prinzip wird beispielsweise durch die in WO 2007 078052 A1 offenbarte Vorrichtung verwirklicht.

Die Patentanmeldung WO2007/078052 A1 offenbart eine Therapievorrichtung, mit einer federgelagerten Therapieplatte und mit zumindest einer Antriebsvorrichtung, die fest mit der Therapieplatte verbunden und dazu ausgebildet ist, eine oszillierende Bewegung in Normalenrichtung der Therapieplatte zu bewirken.

Bei den bekannten Therapievorrichtungen, die in der Regel als kompaktes Standgerät ausgeführt sind (ähnlich einer Personenwaage), sind die Unwuchtmotoren zur Erzeugung der Oszillationsbewegung nicht mehr als einige zehn Zentimeter voneinander entfernt. Die Unwuchtmotoren synchronisieren sich daher bei solch kleinen Vorrichtungen selbsttätig.

Sollen hingegen größere Therapievorrichtungen realisiert werden, wie beispielsweise eine Therapieliege oder noch größere oszillierende Einheiten, wird die o. g. Selbstsynchronisation aufgrund der endlichen Steifigkeit der Therapieplatte zunehmend schwierig, was zu unerwünschten Störbewegungen in Ebenenrichtung führt. Aus diesem Grund müssen bei großen Therapieplatten die Motoren entweder mechanisch oder durch geeignete elektronische Einrichtungen synchronisiert werden.

Motoren, die dieses leisten, sind etwa Dual-Fliehkraftmotoren, die unabhängig von ihrer Drehrichtung unterschiedliche Fliehkräfte aufweisen. Dabei addiert sich in der einen Drehrichtung die Wirkung der Unwuchtteile und in der anderen Drehrichtung wird ein Teil der durch die feste Unwucht erzeugten Fliehkraft durch die bewegliche kompensiert. Siehe bspw. www.enviro.com (Anonym) "Dual-Fliehkraftmotoren DFA 10".

Ausgehend von diesem Stand der Technik ist es daher Aufgabe der vorliegenden Erfindung, eine verbesserte Therapievorrichtung zu schaffen, die auch bei großer Baugröße und vermindertem Aufwand eine Oszillationsbewegung erzeugt, die weniger Störanteile in Ebenenrichtung der Therapiefläche als bisher aufweist.

Diese Aufgabe wird durch eine Therapievorrichtung mit den Merkmalen des unabhängigen Anspruchs 1 gelöst.

Weiterbildungen sind in den Unteransprüchen ausgeführt.

Die erfindungsgemäße Therapievorrichtung weist in einer ersten Ausführungsform eine federgelagerte Therapieplatte und eine Antriebsvorrichtung auf, die fest mit der Therapieplatte verbunden ist. Die Antriebsvorrichtung ist dazu vorgesehen, eine oszillierende Bewegung in Normalenrichtung der Therapieplatte zu bewirken. Die Antriebsvorrichtung weist zwei zueinander gegenläufig betreibbare Unwuchtmotoren mit jeweils einem Motorgehäuse auf, das fest mit der Therapieplatte verbunden ist. Je nachdem, wie groß der Abstand der Motoren zueinander ist, können sie in einem oder zwei Gehäusen untergebracht sein bzw. durch eine oder zwei weitere optische Abdeckungen abgedeckt sein.

Die Therapieplatte weist zumindest eine Sandwichplatte auf, mit der die Antriebsvorrichtung kraftleitend verbunden ist, oder sie ist selbst eine solche. Dabei liegt die Orientierung der Sandwichplattenlagen parallel zur Therapieplattenebene. Von den Lagen der Sandwichplatte ist/sind eine oder mehrere Wabenlage(n) und/oder eine Lage mit wellenförmigem Querschnittsprofil. Weiter verfügt die Therapieplatte vorteilhaft über zumindest zwei Versteifungsstreben oder Versteifungsbänder. Die Antriebsvorrichtung braucht erfindungsgemäß auch keine separate Einrichtung zur Phasensynchronisierung der Unwuchtmotoren.

Unter "kraftleitend" wird hierbei eine mechanische Verbindung verstanden, die hinreichend fest und möglichst spielfrei bzw. spielarm ist, sodass die von der Antriebsvorrichtung erzeugten Kräfte unmittelbar auf die Therapieplatte übertragen werden können. Die Therapieplatte hat an ihrer Oberseite eine Therapiefläche, auf die sich ein Patient beispielsweise setzen, legen oder stellen kann. Durch den Einsatz einer Sandwichplatte in der Therapieplatte wird eine möglichst hohe Steifigkeit bei gleichzeitig geringem Gewicht erreicht. Dies ist vorteilhaft, da auf diese Art sowohl die nötige Antriebsleistung gering gehalten werden kann als auch ungewollte Schwingungsanregung des Gebäudes, in dem die Therapievorrichtung aufgestellt ist, oder von benachbarten Gegenständen, weitgehend verhindert werden können. Ferner ist eine vorbestimmte Minimalsteifigkeit der Therapieplatte notwendig, damit sich diese unter der Einwirkung der Antriebskräfte der Antriebsvorrichtung nicht durchbiegt und so zu einer Verfälschung der rein normal ausgerichteten Bewegung führt. Eine möglichst steife Therapieplatte trägt daher zur "Reinheit" der Oszillationsbewegung in Normalenrichtung bei.

Es ist dabei wichtig, dass die Sandwichplatte zumindest den Bereich der Therapieplatte zwischen Kraftangriffspunkt(en) der Antriebsvorrichtung und den Lagerpunkten, an denen die Federlagerung angreift, abdeckt, oder aber dass in der Therapieplatte vollflächig eine Sandwichstruktur eingebettet ist.

Übliche Oszillationsfrequenzen können beispielsweise im Bereich zwischen 5 und 100 Hz liegen, während die Hübe im Bereich weniger Millimeter bis wenige Zentimeter betragen können, etwa 1 bis 20 mm.

Die federnde Lagerung der Therapieplatte kann beispielsweise über Schraubenfedern erfolgen; es ist aber auch denkbar, Luftfedern oder ein magnetisches Aufhängungssystem einzusetzen. Ausgeschlossen sind jedenfalls Biegefedern oder Blattfedern, insbesondere Spiral- oder Elliptikfedern, insbesondere mit Querverbindungen in einem Federsystem mit mehreren Federn.

In einer weiteren Ausführungsform kann vorteilhafter Weise die Längsausrichtung der Wellen der gewellten Lage der Sandwichplatte in Längsrichtung der Therapieplatte verlaufen.

Mit "längs" wird hierin unabhängig vom Zweck und der räumlichen Anordnung der Therapievorrichtung eine Richtung verstanden, die parallel zu einer langen Seite der Therapieplatte verläuft. Über diese Anordnung wird bezüglich der relevanten Biegeachse (quer zur Längsrichtung der Therapieplatte) ein höheres Flächenträgheitsmoment bereitgestellt, was zu einer höheren Steifigkeit und damit zu insgesamt weniger Durchbiegung um diese Achse führt. Unter "gewellt" und "wellenförmig" werden hierin beliebige Formen verstanden, die wellenartig von einer auf eine andere "Höhe" mäandrieren, beispielsweise sinusförmige, rechteckförmige, zickzackförmige Wellen.

Die Anzahl der Versteifungsstreben oder Versteifungsbänder der Therapievorrichtung kann zusätzlich durch etwa zwei oder mehr diagonal in der Ebene der Therapieplatte verlaufende und sich überkreuzende Versteifungsstreben oder Versteifungsbänder ergänzt werden.

Die Versteifungsstrebe(n) oder (das) Versteifungsband/-bänder können dabei an einer Unterseite der Therapieplatte angeordnet sein und zumindest entlang eines Längenabschnitts mit der Therapieplatte verbunden sein, beispielsweise verklebt.

Die Versteifungsstreben an sich oder zumindest zwei davon können dabei alternativ auch in die Sandwichplatte eingebettet sein. Ferner können auch zusätzlich weitere Versteifungsprofile unter der Therapieplatte vorgesehen sein, mit denen die Motoren vorteilhaft verschraubt oder anderweitig befestigt werden können.

Darüber hinaus ist eine besonders vorteilhafte Ausgestaltung die, bei der die Therapievorrichtung als Therapieliege mit einer länglichen Therapieplatte ausgebildet ist und beispielsweise wenigstens vier Standfüße aufweist, auf denen sich jeweils ein Federelement abstützt, auf dessen dem Standfuß abgewandten Ende die Therapieplatte aufliegt. Optional ist es möglich, dass jeweils zwei Standfüße an einem Kopfende und einem Fußende der Therapieplatte in einem gemeinsamen Standfuß-Gehäuse aufgenommen sind. Alternativ oder zusätzlich kann vorgesehen sein, dass wenigstens einer der Standfüße höhenverstellbar ist, etwa mittels Motor.

Unter "länglich" wird hierbei eine Form der Therapieplatte verstanden, die eine ausgeprägte Längsrichtung hat, sodass es eine kurze und eine lange Seite gibt. Die Abmessungen können dabei vorteilhaft auf menschliche Normmaße abgestimmt sein, beispielsweise 1 m breit und 2,40 m lang, insbesondere aber 0,88 m breit und 2,04 m lang. Die Therapieplatte kann an ihrer Oberseite zusätzlich gepolstert sein und beispielsweise auch eine Heizung aufweisen. Das Standfuß-Gehäuse kann beispielsweise als dünne und leichte Abdeckung aus Kunststoff ausgebildet sein, die die Standfüße schützt und zudem eine ansprechende Gestaltung der Therapievorrichtung ermöglicht.

Ferner ist in einer weiteren Ausführungsform vorgesehen, dass an einer oder beiden Längsseiten der Therapieplatte unter der Therapieplatte wenigstens eine, vorteilhafter Weise zwei in Höhenrichtung zueinander versetzte, Längsverstrebung(en) angeordnet ist/sind. Die Längsverstrebung(en) ist/sind jeweils mit einem der Standfüße am Kopfende und am Fußende der Therapieplatte verbunden.

Es kann dabei vorgesehen sein, dass das Standfuß-Gehäuse entsprechende Durchtrittsöffnungen für die Längsverstrebungen hat, sodass diese mechanisch mit den Standfüßen verbunden werden können. Man könnte sagen, die Standfüße bilden zusammen mit den Längsverstrebungen einen steifen Rahmen. An den Längsverstrebungen können beliebige physiotherapeutische Hilfsmittel, wie etwa eine Leiter, ein Rahmen, eine Treppe, ein Stativ o. ä. angebracht werden. Hierdurch kann die erfindungsgemäße Therapievorrichtung als multifunktionales Therapiesystem eingesetzt werden, das eine Vielzahl weiterer Therapievorrichtungen unnötig macht. So kann eine Brückenkonstruktion über der Therapieplatte auch als Teil eines Schlingentischs eingesetzt werden, wobei hierzu eine Anordnung von Längsverstrebungen an beiden Längsseiten der Therapievorrichtung vorteilhaft ist. Der Schlingentisch kann quasi als Modul angedockt werden. Es können dabei nicht nur die Füße, sondern alle Körperteile eingehängt werden(z. B. Kopf, Arme, Becken, Beine, Füße, auch mehrere davon gleichzeitig). Dies dient zum einen zum hubfreien Training oder zur Ausübung von Traktion auf Gelenke oder die Wirbelsäule (z. B. Zustand nach Bandscheibenvorfall). Der Sinn hierin liegt darin, dass hubfreies Bewegen und Traktionstherapie mit der Stoßwellentherapie kombiniert werden können.

Die therapeutischen Hilfsmittel können zwar direkt an den Längsverstrebungen montiert oder befestigt werden, es ist aber auch möglich, ein Rollensystem an den Längsverstrebungen anzubringen, um die Hilfsmittel auf unterschiedlichen Positionen einsetzen zu können.

Die Verstrebungen sollen so dimensioniert sein, dass sie die bei der Therapie auftretenden Kräfte ohne unzulässig große Verformungen aufnehmen können; sie können beispielsweise als Vollprofil, Hohlprofil oder Platte ausgebildet sein und z. B. aus Aluminium oder Stahl bestehen. Ferner kann vorgesehen sein, an den Längsverstrebungen eine Halterung für ein Bedientablet anzubringen. Bei Nutzung einer Leiter oder Brücke zur Therapie kann in einer besonders vorteilhaften Ausführungsform auch vorgesehen sein, die Leiter oder Brücke auf Rollen auf den Längsverstrebungen verschiebbar zu lagern. Des Weiteren kann an der Längsverstrebung auch eine Halterung für eine Papierrolle vorgesehen werden, sodass die Liege vor jeder Verwendung bequem desinfiziert werden kann.

Darüber hinaus ist es möglich, eine Kopfhalterung vorzusehen, in die der Kopf des Patienten bei Bedarf eingehängt werden kann, sodass dieser nicht der Oszillation ausgesetzt ist. Auf der Therapieplatte und/oder an den Längsverstrebungen kann/können alternativ oder zusätzlich auch eine oder mehrere Fixationsvorrichtungen vorliegen, um Lagerungsmaterial wie Keile oder Klötze an der Therapievorrichtung befestigen zu können.

Die Längsverstrebung(en) kann/können jeweils zumindest eine Kopplungsvorrichtung für vorbestimmte therapeutische Hilfsmittel aufweisen. Bei der Kopplungsvorrichtung kann es sich etwa um eine Längsnut, bevorzugt eine T-Nut oder Schwalbenschwanznut, eine Bohrung, ein Haken und/oder eine Öse handeln. Die Haken, Ösen o. ä. können dabei in Bohrungen, die in vorbestimmten Abständen der Längsverstrebung(en) vorliegen, befestigt werden. Die Erfindung ist jedoch nicht hierauf beschränkt, es können vielmehr andere, dem Fachmann geeignet erscheinende Kopplungsvorrichtungen eingesetzt werden. Es kann aber auch vorgesehen sein, dass die Halterung von außen auf die Längsverstrebung(en) gesteckt ist und längsverschiebbar sowie arretierbar ist.

Die Ausgangswelle der Unwuchtmotoren kann dabei quer zu der Längsrichtung der Therapieplatte ausgerichtet sein, muss aber nicht. Die Unwuchtmotoren können ferner in Längsrichtung der Therapieplatte beabstandet angeordnet sein, wobei der Abstand der Unwuchtmotoren mit der Steifigkeit der Therapieplatte korreliert. Der Abstand kann in einem Ausführungsbeispiel in einem Bereich von 0,0 m, wenn die Motoren unmittelbar aneinander angrenzen, bis 2,0 m liegen, und auch beliebige Maße dazwischen annehmen, etwa 0,5 m, 0,7 m, 1 m oder 1,5 m.

Aufgrund der sehr steifen Therapieplatte ist keine separate Einrichtung zur Phasensynchronisierung der Unwuchtmotoren nötig; sie synchronisieren sich selbsttätig. Dies ist möglich, da eine durch Krafteinwirkung des einen Unwuchtmotors erzeugte Bewegung der Therapieplatte in Normalenrichtung praktisch ohne Zeitverzögerung an den anderen Unwuchtmotor "weitergeleitet" wird. Die Steifigkeit der Therapieplatte wird jedoch stets endlich sein, daher sind dem Effekt der Selbstsynchronisierung Grenzen gesetzt, und zwar hinsichtlich des minimalen Abstands der Unwuchtmotoren. Wird dieser minimale Abstand unterschritten, kommt die Therapieplatte in Eigenresonanz. Optimal für die Selbstsynchronisation ist, die Motoren so nah wie möglich anzuordnen, was auch den Vorteil hat, dass diese mit einer gemeinsamen Abdeckung versehen werden können. Für endlich steife Platten ist daher stets ein Abstandsoptimum zu bestimmen.

Unter "Ausgangswelle" wird hierin die (Antriebs-)Welle des Unwuchtmotors verstanden, auf der die Unwuchtscheiben angeordnet sind; es ist dabei nicht ausgeschlossen, dass der Motor ein Getriebemotor ist, es kann sich aber auch um einen Motor mit Direktantrieb der Ausgangswelle handeln. Insbesondere kann der Unwuchtmotor ein Motor sein, der ein Motorgehäuse mit beidseitigen Wellenausgängen hat, aus denen sich jeweils ein Ausgangswellenstumpf erstreckt, auf denen jeweils zumindest eine Unwuchtscheibe mit exzentrischem Masseschwerpunkt angeordnet ist.

In einer weiteren Ausführungsform kann jeder Unwuchtmotor zumindest zwei angular gegeneinander verdrehbare und arretierbare oder alternativ frei aufhängbare Unwucht-Teilscheiben mit exzentrischem Masseschwerpunkt aufweisen, die auf der Ausgangswelle des jeweiligen Unwuchtmotors angeordnet sind. Zur Winkelverstellung der arretierbaren Unwucht-Teilscheiben kann der Unwuchtmotor ferner eine Rastvorrichtung aufweisen, die vorbestimmte Rastwinkel hat.

Über die angular verstellbaren Unwucht-Teilscheiben kann die effektive Unwucht eingestellt werden; so ist es möglich mit ein und derselben Therapievorrichtung verschiedene Bedarfsfelder zu bedienen, so beispielsweise Altersheime gegenüber Trainingsanwendungen für Leistungssportler. Die Unwucht ist dabei von 0 % (180° Versatz der Unwucht-Teilscheiben) bis 100 % (0° Versatz der Unwucht-Teilscheiben) einstellbar. Ein üblicher, für den Patienten angenehmer Wert beträgt beispielsweise 60 bis 70 %. An gebräuchlichen Winkelpositionen kann die Rastvorrichtung dann jeweils eine Rastposition haben, um diese Positionen schneller zu finden; alternativ oder zusätzlich kann die Rastvorrichtung auch eine vorbestimmte Teilung haben, etwa 10°.

Darüber hinaus kann die Unwucht auch über mehrere Einzelscheiben oder durch eine Umkehrung der Drehrichtung eingestellt bzw. erzielt werden. So kann die Erfindung vorsehen, dass die Unwuchtmotoren paarweise angeordnete Dual-Fliehkraft-Rüttelmotoren sind. Dabei kann sich eine vorbestimmte Anzahl an Scheiben, die auf der Achse montiert sind, in einer bestimmten Drehrichtung bis zu einem versetzbaren bzw. frei einstellbaren Mitnehmer verdrehen und so in einer Drehrichtung eine andere Unwucht erzeugen, als in der Gegenrichtung. So kann, je nachdem, wie viele Scheiben auf der Achse vorgesehen sind, die Unwucht durch die Wahl einer Drehrichtung verstärkt oder abgeschwächt werden. Insbesondere kann ein Paar Dual-Fliehkraft-Rüttelmotoren so angeordnet sein, dass sie in beiden Drehrichtungen mit absolutem Synchronlauf betreibbar sind, insbesondere im Frequenzbereich der Betriebsspannung von 5 Hz bis 100 Hz, vorzugsweise in einem Bereich von 15 Hz bis 70 Hz. Damit wird ein besonders ruhiger und stabiler Antrieb erreicht, der eine optimale Bewegung der Therapieplatte mit Oszillation in Normalenrichtung zur Therapieplatte erlaubt.

Ferner kann die Therapievorrichtung eine Steuerungsvorrichtung aufweisen, die operativ mit der Antriebsvorrichtung verbunden und dazu ausgebildet ist, die Oszillationsfrequenz der Antriebsvorrichtung einzustellen. Optional kann die Steuerungsvorrichtung zumindest eine Steuerungsschnittstelle haben, die bevorzugt zur drahtlosen oder zur kabelgebundenen Kommunikation ausgebildet ist. Die Schnittstelle kann z. B. nach dem WLAN oder Bluetooth®-Standard arbeiten oder eine USB-Schnittstelle sein.

Die Steuerungsvorrichtung weist die Einrichtungen auf, die zum Betrieb der Therapievorrichtung nötig ist, d. h., sie ist primär zur Energiebereitstellung und Drehzahlregelung der Motoren vorgesehen. Sie kann im Wesentlichen aus einer Hauptplatine bestehen, die Frequenzumrichter, Mikrocontroller und eben die genannte Schnittstelle etc. aufweist. Über o. g. Schnittstelle kann die Steuerungsvorrichtung mit einem Endgerät, z. B. einem Tablet, Smartphone, Laptop-PC oder Desktop-PC, verbunden werden, der als Mensch-Maschine-Schnittstelle fungiert und auf dem eine Software ausführbar ist, über die die Funktionen der Therapievorrichtung steuerbar sind. Über die Steuerungsvorrichtung können auf den jeweiligen Patienten abgestimmte Therapieprogramme abgerufen werden, d. h. Abfolgen einer Anregung mit vorbestimmten Oszillationsfrequenzen.

Weiter kann die Steuerungsvorrichtung ein Gehäuse für die Steuerungsvorrichtung und zumindest einen Eingang, etwa einen Energieversorgungsanschluss, und zumindest einen Ausgang, etwa einen Anschluss für die Antriebsvorrichtung, aufweisen. Eingang und Ausgang sind in einem zentralen Verbindungselement zusammengefasst, das an dem Steuerungsvorrichtungs-Gehäuse befestigt ist. Weiter ist am Standfuß-Gehäuse ein Fach, Raum oder Einschub vorhanden, in dem das Gehäuse der Steuerungsvorrichtung entnehmbar aufgenommen ist. In dem Fach, Raum oder Einschub des Standfuß-Gehäu-ses liegt ein mit dem zentralen Verbindungselement des Steuerungsvorrichtungs-Gehäu-ses korrespondierender Verbindungspartner vor, mit dem das zentrale Verbindungselement des Steuerungsvorrichtungs-Gehäuses trennbar verbunden ist.

Beim Herausnehmen des Gehäuses der Steuerungsvorrichtung wird die Verbindung des zentralen Verbindungselements mit seinem Verbindungspartner automatisch getrennt und beim Einsetzen wieder hergestellt. Vorteilhaft wird so ermöglicht, dass die Steuerungsvorrichtung auch von technisch weniger versierten Personen als Ganzes gegen eine funktionstüchtige ausgetauscht werden kann. Als Verbindungselement bzw. dessen korrespondierender Verbindungspartner kommen alle mehrpoligen Stecker-Buchsen Kombinationen in Frage, beispielsweise ISO-Stecker, AMP-Stecker, nur um zwei Beispiele zu nennen.

Darüber hinaus kann die Therapievorrichtung eine kabelgebundene Fernbedienung aufweisen, die operativ mit der Steuerungsvorrichtung verbunden ist. Vorteilhafter Weise kann in einem der Standfuß-Gehäuse eine Klappe, Tür oder Einschub vorgesehen sein, hinter der die kabelgebundene Fernbedienung angeordnet ist. Die kabelgebundene Fernbedienung ist als Not-Steuerungseinheit vorgesehen, die es ermöglicht, auch bei Defekt der Kommunikationsschnittstelle der Steuerungsvorrichtung oder wenn das Endgerät, das die Mensch-Maschine-Schnittstelle bereitstellt, nicht einsatzfähig ist, die Therapievorrichtung zu betreiben und zumindest die Hauptfunktionen "Antrieb ein/aus" und "Oszillationsfrequenz" zu steuern.

Es kann ferner noch ein Anschluss für ein externes Therapiegerät vorgesehen sein, bevorzugt für einen Hand-Stoßwellengenerator, wobei der Anschluss für das externe Therapiegerät operativ mit der Steuerungsvorrichtung verbunden ist und vorbestimmte Betriebsparameter des externen Therapiegeräts bevorzugt über die Steuerungsvorrichtung steuerbar sind. Auch die Verbindung des Anschlusses für das externe Therapiegerät und der Steuerungsvorrichtung kann dabei über das zentrale Verbindungselement des Steuerungsvorrichtungs-Gehäuses verbunden/getrennt werden. Der Anschluss für das externe Therapiegerät kann dabei an einem ergonomisch günstigen Punkt der Therapievorrichtung, beispielweise an dem Standfuß-Gehäuse oder auf einem nicht genutzten Bereich an der Oberseite der Therapieplatte angeordnet sein. Bei dem externen Therapiegerät kann es sich alternativ zu dem genannten Stoßwellengenerator auch um eine Vorrichtung zur Wärmebehandlung, Ultraschallbehandlung, Massage handeln. Unter "Betriebsparameter" kann je nach eingesetztem externen Therapiegerät eine Leistung, eine Antriebsdrehzahl oder die Frequenz einer erzeugten Schwingung verstanden werden.

Schließlich kann die Therapievorrichtung eine Datenträger-Lesevorrichtung, etwa einen USB-Steckplatz, einen Chipkartenleser, ein NFC-Lesegerät, aufweisen, die kommunikativ mit der Steuerungsvorrichtung verbunden ist. Mit der Datenträger-Lesevorrichtung können beispielsweise Therapieprogrammdaten in die Steuerungsvorrichtung geladen werden. Unter Therapieprogrammdaten sind zumindest die Abfolgen und Dauern des Betriebs der Antriebsvorrichtung und deren Drehzahl zu verstehen. In den Therapieprogrammdaten können aber auch Informationen zur Beleuchtung und beispielsweise bei der Therapie abzuspielenden Audiodaten hinterlegt sein. Ähnlich wie bei einem Fitnessstudio mit automatisiert personalisierbaren Trainingsgeräten kann die erfindungsgemäße Therapievorrichtung so möglichst schnell auf den nächsten Patient eingestellt werden.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Die Figuren sind lediglich schematische Darstellungen von Ausführungsbeispielen der Erfindung.

Dabei zeigen:
- Fig. 1: eine Seitenansicht der Therapievorrichtung,
- Fig. 2: eine Detail-Schnittansicht der Therapieplatte der Therapievorrichtung,
- Fig. 3: eine Seitenansicht einer weiteren Ausführungsform der Therapievorrichtung,
- Fig. 4: eine perspektivische Ansicht einer weiteren Ausführungsform der Therapievorrichtung.

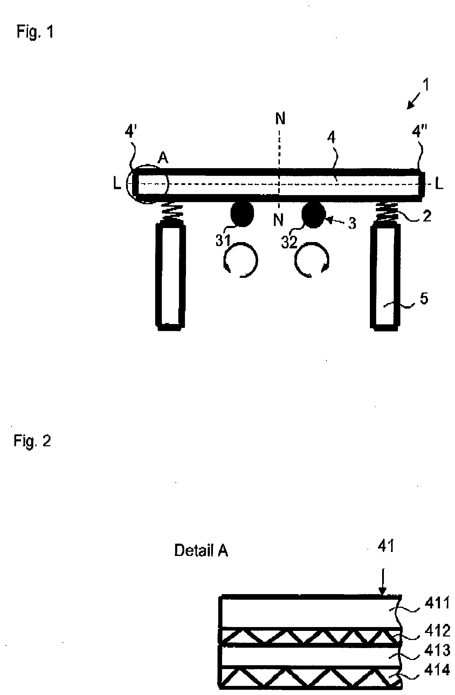

Als Beispiel für eine erfindungsgemäße Therapievorrichtung ist in **Fig. 1** die Therapieliege 1 gezeigt, auf die sich ein Patient legen kann (er kann aber darauf auch problemlos knien, stehen oder sitzen). Sie hat eine Therapieplatte 4, die über Schraubenfedern 2 auf Standfüßen 5 gelagert ist. Mit der Unterseite der Therapieplatte 4 ist die Antriebsvorrichtung 3 fest verbunden, etwa verschraubt. Bei der Antriebsvorrichtung 3 handelt es sich um zwei gegenläufig betreibbare Unwuchtmotoren 31,32, deren Drehrichtung durch die Pfeile symbolisiert wird. Die Unwuchtmotoren 31,32 sind beide fest mit der Therapieplatte 4 verbunden, sodass die durch die exzentrischen Masseschwerpunkte erzeugten Kräfte auf die Therapieplatte 4 übertragen werden. In der Folge wird eine Oszillationsbewegung in Normalenrichtung N der Therapieplatte 4 bewirkt. Da die Unwuchtmotoren 31,32 gegenläufig rotieren, muss keine Führung o. ä. in Normalenrichtung vorgesehen werden; die Bewegungskomponenten in Ebenenrichtung der Therapieplatte heben sich gegenseitig auf und die Bewegungskomponenten in Normalenrichtung addieren sich, so dass eine ideal in Normalenrichtung gerichtete Oszillationsbewegung der Therapieplatte 4 erreicht wird. Der Grad der Schwingungsanregung kann durch angular verstellbare und arretierbare Unwucht-Teilscheiben, die auf den Ausgangswellen der Unwuchtmotoren 31,32 angeordnet sind, eingestellt werden, wobei ein Versatz von 0° zu maximaler Anregung führt.

Erfindungsgemäß kann auch auf Einrichtungen zur Phasensynchronisation der beiden Unwuchtmotoren 31,32 verzichtet werden; die Unwuchtmotoren 31,32 synchronisieren sich vielmehr selbsttätig, da beide fest mit der Therapieplatte 4 verbunden, etwa verschraubt, sind.

Um eine möglichst effektive Synchronisation und damit möglichst wenig Bewegungsanteile in anderen, von der Normalenrichtung abweichenden Richtungen zu erreichen, ist die Therapieplatte 4 erfindungsgemäß als steife Sandwichplatte 41 ausgebildet, was in **Fig. 2** mit dem Schichtaufbau gezeigt ist.

Die Sandwichplatte 41 besteht in dem gezeigten Ausführungsbeispiel aus vier Lagen 411,412,413,414, von denen zwei Lagen gewellte Lagen 412,414 sind. Neben der gezeigten Zickzackform der Welllagen 412,414 sind auch andere Wellenformen, etwa sinusförmig, möglich. Besonders vorteilhaft sind die Wellen 412',414' mit ihrer Längsrichtung in Längsrichtung der Therapieplatte 4 ausgerichtet, sodass eine maximale Biegesteifigkeit erreicht werden kann. Bei den Lagen 411 und 413 kann es sich beispielsweise um massive Platten aus Aluminium, Kunststoff, Holz, insbesondere Spanplatten bzw. Schichtholz, handeln. Alternativ oder zusätzlich, figurativ jedoch nicht gezeigt, können zwischen den einzelnen Lagen auch (Hart-)Schaumlagen vorgesehen sein. Der Aufbau der Sandwichplatte 41 kann auch ein Lagenaufbau ohne Wellen und Waben sein.

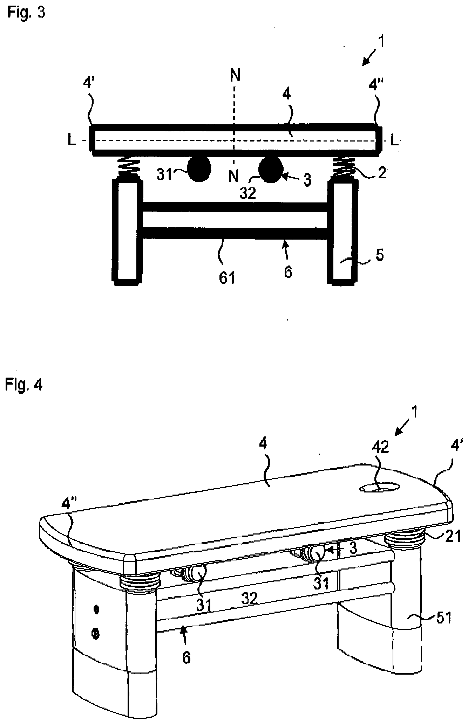

Die in **Fig. 3** gezeigte Ausführungsform entspricht der Ausführungsform der **Fig. 1**, abgesehen von den Längsverstrebungen 6, die in **Fig. 1** nicht vorliegen. Die Längsverstrebungen 6 sind unter der Therapieplatte 4 angeordnet und jeweils mit einem Standfuß am Kopfende 4' und einem Standfuß am Fußende 4" der Therapieplatte 4 verbunden. Es hat hier pro Längsseite zwei in Höhenrichtung versetzt angeordnete Längsverstrebungen 6. An den Längsverstrebungen 6 können physiotherapeutische Hilfsmittel wie Leitern, Brücken, Treppen, Bänder usw. befestigt werden. Die erfindungsgemäße Therapievorrichtung wird damit zum multifunktionalen Therapiesystem weitergebildet. Es ist also mit der erfindungsgemäßen Therapievorrichtung bzw. Therapieliege 1 nicht nur eine niederfrequente Stoßwellenbehandlung möglich, sondern es können mit ein und derselben Vorrichtung auch "herkömmliche" Therapien durchgeführt werden. Die Profile der Längsverstrebungen 6 werden geeigneter Weise so dimensioniert, dass sie die Therapiekräfte ableiten können ohne sich merklich zu verformen. Um die therapeutischen Hilfsmittel mit den Längsverstrebungen 6 zu koppeln, ist hier eine in Längsrichtung verlaufende Nut (z. B. T-Nut) vorgesehen, in die bestimmte Ösen, Haken o. ä. eingehängt werden können.

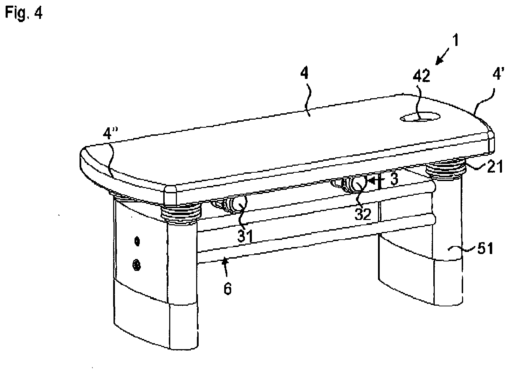

Bei der in **Fig. 4** gezeigten Weiterbildung sind die Standfüße am Kopf- und Fußende der Therapieplatte 4 jeweils in einem gemeinsamen Standfuß-Gehäuse 51 aufgenommen, das hier als dekorative Abdeckung ausgebildet ist. Die Federn sind in der Abbildung nicht zu sehen; die Federn befinden sich unter den Faltenbalgen 21. Die Therapieplatte 4 ist an ihrem Kopfende 4' und Fußende 4" jeweils sanft verrundet und im Kopfbereich liegt eine Aussparung 42 vor, in die der Patient seinen Kopf legen kann, um auch auf dem Bauch liegend angenehm therapiert werden zu können. Die Oberseite der Therapieplatte 4, die eigentliche Therapiefläche, kann zudem beheizt und/oder gepolstert sein. Die Standfuß-Gehäuse 51 können, was figurativ nicht gezeigt ist, eine Klappe oder Tür haben, hinter der die Kabelfernbedienung aufgenommen sein kann.

Gemäß einer figurativ nicht gezeigten Weiterbildung ist vorgesehen, dass die Therapievorrichtung eine oder mehrere Beleuchtungsvorrichtung(en) aufweist, bevorzugt farbeinstellbare Beleuchtungsvorrichtungen, etwa eine Mehrzahl farbeinstellbarer LEDs, die unter der Therapieplatte angeordnet sind. Die farbeinstellbaren Beleuchtungsvorrichtungen können zur Farbeinstellung operativ mit einem Beleuchtungssteuerungsgerät und/oder der Steuerungsvorrichtung verbunden sein. Alternativ oder zusätzlich kann die Therapievorrichtung eine Audiowiedergabevorrichtung aufweisen, die zur Wiedergabe von Audiodaten operativ mit der Steuerungsvorrichtung und/oder einer separaten Audiospeichervorrichtung verbunden ist, wodurch möglich ist, dem Patient individuelle Musik zur Unterstützung des Therapieziels und/oder Anweisungen einzuspielen, etwa Entspannungsmusik. Auch eine visuelle und akustische Einführung in die Therapievorrichtung kann eingespielt werden (im Sinne einer Bedienungsanleitung).

Die Steuerungsvorrichtung kann hierbei gewissermaßen die Funktion einer Head-Unit übernehmen, von der alle therapierelevanten Funktionen bedient werden, d. h., die Antriebsvorrichtung gesteuert wird, Beleuchtungsintensität, Beleuchtungsfarbe, Musik bzw. bildunterstützte und/oder optisch erfassbare Anweisungen in Abhängigkeit eines individuellen Therapieplans einstellbar sind.

## Patentansprüche

1. Therapievorrichtung,
- mit einer federgelagerten Therapieplatte (4) und
- mit zumindest einer Antriebsvorrichtung (3), die fest mit der Therapieplatte (4) verbunden und dazu ausgebildet ist, eine oszillierende Bewegung in Normalenrichtung (N) der Therapieplatte (4) zu bewirken, und wobei die Antriebsvorrichtung (3) zwei zueinander gegenläufig betreibbare Unwuchtmotoren (31,32) mit jeweils einem Motorgehäuse aufweist, das fest mit der Therapieplatte (4) verbunden ist,
**dadurch gekennzeichnet, dass**
die Therapieplatte (4)
- zumindest zwei Versteifungsstreben oder Versteifungsbänder aufweist,
- zumindest eine Sandwichplatte (41) aus mehreren Lagen aufweist oder ist, mit der die Antriebsvorrichtung (3) kraftleitend verbunden ist, wobei die Orientierung der Lagen der Sandwichplatte parallel zu einer Therapieplattenebene verläuft, die Sandwichplatte (41) mehrere Lagen (411,412,413,414) aufweist, von denen zumindest eine Lage eine Wabenlage ist und/oder ein wellenförmiges Querschnittsprofil (412,414) aufweist, und wobei die Antriebsvorrichtung (3) keine separate Einrichtung zur Phasensynchronisierung der Unwuchtmotoren (31,32) aufweist.

2. Therapievorrichtung nach Anspruch 1,
**dadurch gekennzeichnet, dass**
eine Längsausrichtung der Wellen (412',414') der Wabenlage der Sandwichplatte (41) und/oder der Lage mit dem wellenförmigen Querschnittsprofil (412,414) der Sandwichplatte (41) in Längsrichtung der Therapieplatte (4) verläuft.

3. Therapievorrichtung nach Anspruch 1 oder 2,
**dadurch gekennzeichnet, dass**
- zumindest zwei diagonal in der Ebene der Therapieplatte (4) verlaufende und sich überkreuzende Versteifungsstreben oder Versteifungsbänder vorgesehen sind, und/oder
wobei besonders bevorzugt die Versteifungsstrebe(n) oder Versteifungsbänder an der Unterseite der Therapieplatte (4) angeordnet und zumindest entlang eines Längenabschnitts mit der Therapieplatte (4) verbunden ist/sind.

4. Therapievorrichtung nach zumindest einem der Ansprüche 1 bis 3,
**dadurch gekennzeichnet, dass**
- die Therapievorrichtung als Therapieliege (1) mit einer länglichen Therapieplatte (4) ausgebildet ist und/oder
- zumindest vier Standfüße (5) aufweist, auf denen sich jeweils ein Federelement (2) abstützt, auf dessen dem Standfuß (5) abgewandten Ende die Therapieplatte (4) aufliegt,
wobei bevorzugt
- jeweils zwei Standfüße (5) an einem Kopfende (4') und einem Fußende (4") der Therapieplatte (4) in einem gemeinsamen Standfuß-Gehäuse (51) aufgenommen sind und/oder
- wobei bevorzugt zumindest einer der Standfüße (5) höhenverstellbar ist, bevorzugt motorisch höhenverstellbar.

5. Therapievorrichtung nach Anspruch 4,
**dadurch gekennzeichnet, dass**
an zumindest einer Längsseite der Therapieplatte (4) unter der Therapieplatte (4) zumindest eine, bevorzugt zwei in Höhenrichtung zueinander versetzte, Längsverstrebung(en) (6) angeordnet ist/sind, die jeweils mit einem der Standfüße (5) am Kopfende (4') und am Fußende (4") der Therapieplatte (4) verbunden ist/sind.

6. Therapievorrichtung nach Anspruch 5,
**dadurch gekennzeichnet, dass**
die Längsverstrebung (6) zumindest eine Kopplungsvorrichtung (61) für vorbestimmte therapeutische Hilfsmittel aufweist,
wobei die zumindest eine Kopplungsvorrichtung (61) bevorzugt eine Längsnut (61), besonders bevorzugt eine T-Nut oder Schwalbenschwanznut, eine Bohrung, ein Haken und/oder eine Öse ist.

7. Therapievorrichtung nach zumindest einem der Ansprüche 1 bis 6,
**dadurch gekennzeichnet, dass**
jeder Unwuchtmotor (31,32) zumindest zwei angular gegeneinander verdrehbare, frei aufhängbare oder arretierbare Unwucht-Teilscheiben mit exzentrischem Masseschwerpunkt aufweist, die auf der Ausgangswelle angeordnet sind,
wobei bevorzugt die arretierbaren Unwucht-Teilscheiben zur Winkelverstellung eine Rastvorrichtung aufweisen, die vorbestimmte Rastwinkel hat.

8. Therapievorrichtung nach zumindest einem der Ansprüche 1 bis 6,
**dadurch gekennzeichnet, dass**
- die Unwuchtmotoren (31,32) paarweise angeordnete Dual-Fliehkraft-Rüttelmotoren sind, insbesondere ein Paar Dual-Fliehkraft-Rüttelmotoren (31,32), die so angeordnet sind, dass sie in beiden Drehrichtungen mit absolutem Synchronlauf betreibbar sind, insbesondere im Frequenzbereich der Betriebsspannung von 20 Hz bis 60 Hz, und/oder
- wobei bevorzugt eine Ausgangswelle der Unwuchtmotoren (31,32) jeweils quer zu der Längsrichtung (L) der Therapieplatte (4) ausgerichtet ist und/oder
- die Unwuchtmotoren (31,32) in Längsrichtung der Therapieplatte (4) angeordnet sind
und wobei insbesondere ein Abstand der Unwuchtmoren (31,32) voneinander mit der Steifigkeit der Therapieplatte (4) korreliert,
wobei bevorzugt
ein Abstand der Unwuchtmotoren von 0 m bis 2 m beträgt.

9. Therapievorrichtung nach zumindest einem der Ansprüche 1 bis 8,
**dadurch gekennzeichnet, dass**
- die Therapievorrichtung eine Steuerungsvorrichtung aufweist, die operativ mit der Antriebsvorrichtung (3) verbunden und dazu ausgebildet ist, die Oszillationsfrequenz der Antriebsvorrichtung (3) einzustellen,
- wobei bevorzugt die Steuerungsvorrichtung zumindest eine Steuerungsschnittstelle aufweist, die bevorzugt zur drahtlosen oder zur kabelgebundenen Kommunikation ausgebildet ist.

10. Therapievorrichtung nach Anspruch 9,
**dadurch gekennzeichnet, dass**
die Steuerungsvorrichtung
- ein Gehäuse aufweist und
- zumindest einen Eingang, bevorzugt einen Energieversorgungsanschluss, und zumindest einen Ausgang, bevorzugt einen Anschluss für die Antriebsvorrichtung (3), aufweist,
- wobei Eingang und Ausgang in einem zentralen Verbindungselement zusammengefasst sind, das an dem Gehäuse der Steuerungsvorrichtung befestigt ist, und
- das Standfuß-Gehäuse (51) ein Fach, Raum oder Einschub aufweist, in dem das Gehäuse der Steuerungsvorrichtung entnehmbar aufgenommen ist,
- wobei in dem Fach, Raum oder Einschub des Standfuß-Gehäuses (51) ein mit dem zentralen Verbindungselement des Gehäuses der Steuerungsvorrichtung korrespondierender Verbindungspartner vorliegt, mit dem das zentrale Verbindungselement des Steuerungsvorrichtungs-Gehäuses lösbar verbunden ist.

11. Therapievorrichtung nach Anspruch 9 oder 10,
**dadurch gekennzeichnet, dass**
die Therapievorrichtung eine kabelgebundene Fernbedienung aufweist, die operativ mit der Steuerungsvorrichtung verbunden ist,
wobei bevorzugt in zumindest einem der Standfuß-Gehäuse (51) eine Klappe, Tür und/oder Einschub vorgesehen ist, hinter der die kabelgebundene Fernbedienung angeordnet ist.

12. Therapievorrichtung nach zumindest einem der Ansprüche 9 bis 11,
**dadurch gekennzeichnet, dass**
die Therapievorrichtung zumindest einen Anschluss für ein externes Therapiegerät aufweist, bevorzugt für einen Hand-Stoßwellengenerator, wobei der Anschluss für das externe Therapiegerät operativ mit der Steuerungsvorrichtung verbunden ist und vorbestimmte Betriebsparameter des externen Therapiegeräts bevorzugt über die Steuerungsvorrichtung steuerbar sind.

13. Therapievorrichtung nach zumindest einem der Ansprüche 9 bis 12,
**dadurch gekennzeichnet, dass**
die Therapievorrichtung eine Datenträger-Lesevorrichtung, bevorzugt einen USB-Steckplatz, einen Chipkartenleser, ein NFC-Lesegerät, aufweist, die kommunikativ mit der Steuerungsvorrichtung verbunden ist, wobei mittels der Datenträger-Lesevorrichtung bevorzugt Therapieprogrammdaten in die Steuerungsvorrichtung ladbar sind.

## Claims

1. A therapy device,
- comprising a spring-supported therapy plate (4) and
- comprising at least one drive device (3), which is firmly connected to the therapy plate (4) and which is formed to effect an oscillating movement in the normal direction (N) of the therapy plate (4), and wherein the drive device (3) has two unbalance motors (31, 32), which can be operated in opposite directions to one another, each comprising a motor housing, which is firmly connected to the therapy plate (4),
**characterised in that**
the therapy plate (4)
- has at least two reinforcement struts or reinforcement straps,
- has or is at least one sandwich plate (41) of several layers, to which the drive device (3) is connected so as to guide a force, wherein the orientation of the layers of the sandwich plate runs parallel to a therapy plate plane, the sandwich plate (41) has several layers (411, 412, 413, 414), at least one layer of which is a honeycomb layer and/or has an undulating cross-sectional profile (412, 414), and wherein the drive device (3) does not have a separate unit for the phase synchronisation of the unbalance motors (31, 32).

2. The therapy device according to claim 1,
**characterised in that**
a longitudinal alignment of the waves (412', 414') of the honeycomb layer of the sandwich plate (41) and/or of the layer comprising the undulated cross-sectional profile (412, 414) of the sandwich plate (41) runs in the longitudinal direction of the therapy plate (4).

3. The therapy device according to claim 1 or 2,
**characterised in that**
- at least two reinforcement struts or reinforcement straps, which run diagonally in the plane of the therapy plate (4) and which cross one another, are provided,
and/or
wherein the reinforcement strut(s) or reinforcement straps are particularly preferably arranged on the underside of the therapy plate (4) and is/are connected to the therapy plate (4) at least along a longitudinal section.

4. The therapy device according to at least any one of claims 1 to 3,
**characterised in that**
- the therapy device is formed as therapy couch (1) comprising an elongated therapy plate (4) and/or
- has at least four feet (5), on which a spring element (2) is in each case supported, on the end of which, which faces away from the foot (5), the therapy plate (4) bears,
wherein preferably
- two feet (5) are in each case received at a top end (4') and a foot end (4") of the therapy plate (4) in a common foot housing (51), and/or
- wherein preferably at least one of the feet (5) is height-adjustable, preferably height-adjustable by motor.

5. The therapy device according to claim 4,
**characterised in that**
at least one, preferably two longitudinal strut member(s) (6), which is/are offset relative to one another in the height direction and which is/are in each case connected to one of the feet (5) at the top end (4') and at the foot end (4") of the therapy plate (4), is/are arranged on at least one longitudinal side of the therapy plate (4) below the therapy plate (4).

6. The therapy device according to claim 5,
**characterised in that**
the longitudinal strut member (6) has at least one coupling device (61) for predetermined therapeutic aids,
wherein the at least one coupling device (61) is preferably a longitudinal groove (61), particularly preferably a T-groove or dovetail groove, a bore, a hook and/or an eye.

7. The therapy device according to at least any one of claims 1 to 6,
**characterised in that**
each unbalance motor (31, 32) has at least two unbalance partial disks comprising eccentric centre of mass, which can be rotated angularly against one another, can be suspended freely or locked, and which are arranged on the output shaft,
wherein, for the angular adjustment, the lockable unbalance partial disks preferably have a locking device, which has predetermined locking angles.

8. The therapy device according to at least any one of claims 1 to 6,
**characterised in that**
- the unbalance motors (31, 32) are dual centrifugal force vibrating motors, which are arranged in pairs, in particular a pair of dual centrifugal force vibrating motors (31, 32), which are arranged such that they can be operated in both directions of rotation with absolute synchronous run, in particular in the frequency range of the operating voltage of from 20 Hz to 60 Hz,
and/or
- wherein an output shaft of the unbalance motors (31, 32) is in each case preferably aligned transversely to the longitudinal direction (L) of the therapy plate (4) and/or
- the unbalance motors (31, 32) are arranged in the longitudinal direction of the therapy plate (4),
and wherein in particular a distance of the unbalance motors (31, 32) from one another correlates to the stiffness of the therapy plate (4),
wherein preferably
a distance of the unbalance motors is from 0 m to 2 m.

9. The therapy device according to at least any one of claims 1 to 8,
**characterised in that**
- the therapy device has a control device, which is operatively connected to the drive device (3) and is formed to set the oscillation frequency of the drive device (3),
- wherein the control device preferably has at least one control interface, which is preferably formed for the wireless or for the wired communication.

10. The therapy device according to claim 9,
**characterised in that**
the control device
- has a housing and
- at least one input, preferably an energy supply connection, and at least one output, preferably a connection for the drive device (3),
- wherein input and output are combined in a central connecting element, which is fastened to the housing of the control device, and
- the foot housing (51) has a compartment, space or slot, in which the housing of the control device is received so as to be capable of being removed,
- wherein a connecting partner, which corresponds to the central connecting element of the housing of the control device and to which the central connecting element of the control device housing is releasably connected, is present in the compartment, space or slot of the foot housing (51).

11. The therapy device according to claim 9 or 10,
**characterised in that**
the therapy device has a wired remote control, which is operatively connected to the control device,
wherein a flap, door and/or slot, behind which the wired remote control is arranged, is preferably provided in at least one of the feet housings (51).

12. The therapy device according to at least any one of claims 9 to 11,
**characterised in that**
the therapy device has at least one connection for an external therapy unit, preferably for a hand shock wave generator, wherein the connection for the external therapy unit is operatively connected to the control device, and predetermined operating parameters of the external therapy unit can preferably be controlled via the control device.

13. The therapy device according to at least any one of claims 9 to 12,
**characterized in that**
the therapy device has a data carrier reading device, preferably a USB slot, a chipcard reader, an NFC reading apparatus, which is communicatively connected to the control device, wherein therapy program data can preferably be loaded into the control device by means of the data carrier reading device.

## Revendications

1. Dispositif thérapeutique,
- avec une plaque thérapeutique montée sur ressorts (4) et
- avec au moins un dispositif d'entraînement (3) qui est fixé à la plaque thérapeutique (4) et conçu pour induire un mouvement oscillant dans la direction normale (N) de la plaque thérapeutique (4) et le dispositif d'entraînement (3) présentant deux moteurs à balourds (31, 32) tournant en sens contraire l'un par rapport à l'autre avec respectivement un carter de moteur qui fixé à la plaque thérapeutique (4),
**caractérisé en ce que**
la plaque thérapeutique (4)
- présente au moins deux entretoises de renforcement ou bandes de renforcement,
- présente ou est au moins une plaque sandwich (41) composée de plusieurs couches, à laquelle le dispositif d'entraînement (3) est relié par conduction de force, l'orientation des couches de la plaque sandwich étant parallèle à un niveau de la plaque thérapeutique, la plaque sandwich (41) présentant plusieurs couches (411, 412, 413, 414), parmi lesquelles au moins une couche est une couche en gauffres et/ou présente un profil de coupe transversale ondulé (412, 414), et le dispositif d'entraînement (3) ne présentant pas de dispositif distinct pour la synchronisation de phases des moteurs à balourds (31, 32).

2. Dispositif thérapeutique conformément à la revendication n°1,
**caractérisé en ce que**
une orientation longitudinale des ondes (412', 414') de la couche en gauffres de la plaque sandwich (41) et/ou de la couche avec le profil de coupe transversale ondulé (412, 414) de la plaque sandwich (41) s'opère dans le sens longitudinal de la plaque thérapeutique (4).

3. Dispositif thérapeutique conformément à la revendication n°1 ou n°2,
**caractérisé en ce que**
- au moins deux entretoises de renforcement ou bandes de renforcement, en diagonale au niveau de la plaque thérapeutique (4) et se croisant, sont prévues,
et/ou
en particulier préférablement l'(les) entretoise(s) de renforcement ou bandes de renforcement étant disposée(s) dans la partie inférieure de la plaque thérapeutique (4) et étant reliée(s) à la plaque thérapeutique (4) au moins le long d'une section de longueur.

4. Dispositif thérapeutique conformément au moins à l'une des revendications n°1 à n°3,
**caractérisé en ce que**
- le dispositif thérapeutique est conçu comme table thérapeutique (1) avec une plaque thérapeutique (4) de forme oblongue et/ou
- présente au moins quatre supports (5) sur lesquels s'appuie respectivement un élément de suspension (2), à l'extrémité opposée au support (5) duquel repose la plaque thérapeutique (4),
préférablement
- deux supports (5) étant respectivement logés à une extrémité de tête (4') et à une extrémité de pied (4") de la plaque thérapeutique (4) dans un boîtier de supports commun (51) et/ou
- préférablement au moins l'un des supports (5) étant réglable en hauteur, préférablement réglable en hauteur par moteur.

5. Dispositif thérapeutique conformément à la revendication n°4,
**caractérisé en ce que**
sur au moins un côté longitudinal de la plaque thérapeutique (4) sous la plaque thérapeutique (4) est/sont disposée(s) au moins une, préférablement deux entretoise(s) longitudinale(s) (6) décalée(s) dans le sens de la hauteur l'une par rapport à l'autre, laquelle/lesquelles est/sont reliée(s) respectivement à l'extrémité de tête (4') et à l'extrémité de pied (4") de la plaque thérapeutique (4) avec l'un des supports (5).

6. Dispositif thérapeutique conformément à la revendication n°5,
**caractérisé en ce que**
l'entretoise longitudinale (6) présente au moins un dispositif d'accouplement (61) pour des équipements thérapeutiques prédéterminés,
au moins un dispositif d'accouplement (61) étant préférablement une rainure longitudinale (61), en particulier préférablement une rainure en T ou une rainure en queue d'aronde, un trou, un crochet et/ou un anneau.

7. Dispositif thérapeutique conformément au moins à l'une des revendications n°1 à n°6,
**caractérisé en ce que**
chaque moteur à balourds (31, 32) présente au moins deux disques partiels à balourds avec centre de gravité excentrique, pouvant tourner angulairement l'un contre l'autre, pouvant être librement suspendus ou pouvant être bloqués, lesquels sont disposés sur l'arbre de sortie,
préférablement les disques partiels à balourds pouvant être bloqués présentant un dispositif d'enclenchement pour le réglage angulaire, lequel a des angles d'enclenchement prédéterminés.

8. Dispositif thérapeutique conformément au moins à l'une des revendications n°1 à n°6,
**caractérisé en ce que**
- les moteurs à balourds (31, 32) sont des moteurs vibrants à double force centrifuge disposés par paire, en particulier une paire de moteurs vibrants à double force centrifuge (31, 32) qui sont disposés de façon à pouvoir être exploités dans les deux sens de rotation avec un fonctionnement synchrone absolu, en particulier dans la gamme de fréquences de la tension de service de 20 Hz à 60 Hz,
et/ou
- préférablement un arbre de sortie des moteurs à balourds (31, 32) étant respectivement aligné transversalement au sens longitudinal (L) de la plaque thérapeutique (4) et/ou
- les moteurs à balourds (31, 32) étant disposés dans le sens longitudinal de la plaque thérapeutique (4)
et en particulier une distance entre les moteurs à balourds (31, 32) étant en corrélation avec la rigidité de la plaque thérapeutique (4),
préférablement
une distance entre les moteurs à balourds (31, 32) étant de 0 m à 2 m.

9. Dispositif thérapeutique conformément au moins à l'une des revendications n°1 à n°8,
**caractérisé en ce que**
- le dispositif thérapeutique présente un dispositif de commande qui est relié opérationnellement au dispositif d'entraînement (3) et conçu pour adapter la fréquence d'oscillation du dispositif d'entraînement (3),
- préférablement le dispositif de commande présentant au moins une interface de commande qui est conçue préférablement pour une communication avec ou sans fil.

10. Dispositif thérapeutique conformément à la revendication n°9,
**caractérisé en ce que**
le dispositif de commande
- présente un boîtier et
- au moins une entrée, préférablement une prise d'alimentation en énergie et au moins une sortie, préférablement une prise pour le dispositif d'entraînement (3),
- l'entrée et la sortie étant regroupées dans un élément de liaison central qui est fixé sur le boîtier du dispositif de commande et
- le boîtier de supports (51) présentant un compartiment, un espace ou un tiroir dans lequel le boîtier du dispositif de commande est logé de façon amovible,
- un partenaire de liaison correspondant à l'élément de liaison central du boîtier du dispositif de commande, auquel l'élément de liaison central du boîtier du dispositif de commande est relié de façon amovible, se trouvant dans le compartiment, l'espace ou le tiroir du boîtier de supports (51).

11. Dispositif thérapeutique conformément à la revendication n°9 ou n°10,
**caractérisé en ce que**
le dispositif thérapeutique présente une télécommande avec fil qui est reliée opérationnellement au dispositif de commande,
préférablement dans au moins l'un des boîtiers de supports (51) est prévu un clapet, une porte et/ou un tiroir, derrière lequel est disposée la télécommande avec fil.

12. Dispositif thérapeutique conformément au moins à l'une des revendications n°9 à n°11,
**caractérisé en ce que**
le dispositif thérapeutique présente au moins une prise pour un appareil thérapeutique externe, préférablement pour un générateur d'ondes de choc manuel, la prise pour l'appareil thérapeutique externe étant reliée opérationnellement au dispositif de commande et les paramètres d'exploitation prédéterminés de l'appareil thérapeutique externe pouvant être contrôlés préférablement par le dispositif de commande.

13. Dispositif thérapeutique conformément au moins à l'une des revendications n°9 à n°12,
**caractérisé en ce que**
le dispositif thérapeutique présente un appareil de lecture de supports de données, préférablement un port USB, un lecteur de cartes à puce, un lecteur NFC, qui est relié du point de vue de la communication au dispositif de commande, des données du programme thérapeutique pouvant être préférablement chargées dans le dispositif de commande au moyen de l'appareil de lecture de supports de données.
